(19) Europäisches Patentamt
European Patent Office
Office européen des brevets

(11) Publication number: **0 468 077 A1**

(12) **EUROPEAN PATENT APPLICATION**

(21) Application number: **90114400.6**

(51) Int. Cl.$^5$: **A61K 6/00**, C08G 18/80

(22) Date of filing: **26.07.90**

(43) Date of publication of application:
**29.01.92 Bulletin 92/05**

(84) Designated Contracting States:
**CH DE FR GB LI LU**

(71) Applicant: **MITSUBISHI RAYON CO., LTD.**
**3-19, Kyobashi 2-chome Chuo-Ku**
**Tokyo 104(JP)**

(72) Inventor: **Mukai, Nobuhiro, Central Research Laboratories**
**Mitsubishi Rayon Co., Ltd., 20-1, Miyuki-cho**
**Otake-shi, Hiroshima 739-06(JP)**
Inventor: **Ige, Hitoshi, Central Research Laboratories**
**Mitsubishi Rayon Co., Ltd., 20-1, Miyuki-cho**
**Otake-shi, Hiroshima 739-06(JP)**
Inventor: **Makino, Takayuki, Central Research Laboratories**
**Mitsubishi Rayon Co., Ltd., 20-1, Miyuki-cho**
**Otake-shi, Hiroshima 739-06(JP)**
Inventor: **Atarashi, Junko, Central Research Laboratories**
**Mitsubishi Rayon Co., Ltd., 20-1, Miyuki-cho**
**Otake-shi, Hiroshima 739-06(JP)**

(74) Representative: **Wächtershäuser, Günter, Dr.**
**Tal 29**
**W-8000 München 2(DE)**

(54) **Dental adhesive.**

(57) A dental adhesive consisting essentially of a combination of:
(A) at least one trifunctional isocyanate compound, or a composition having an inert diluent incorporated to such isocyanate compound, and
(B) a mixture comprising, as the main components, (a) at least one radical polymerizable unsaturated monomer, (b) a radical polymerization initiator, and (c) a polymerizable phosphate compound.

EP 0 468 077 A1

The present invention relates to a dental adhesive having excellent adhesiveness and little irritating properties, which is useful for bonding restorative materials (such as metallic materials, organic polymeric materials and ceramic materials) to living tooth tissues.

Heretofore, in the field of dental materials, various metallic materials (such as gold, silver, platinum, alloys and amalgams), organic polymeric materials (such as polymethyl methacrylate, polycarbonate, polyfunctional vinyl cured products, and composite resins) and ceramic materials (such as porcelain and implant materials) have been used for restoring teeth, for example, for restoring dental carious (so-called decayed tooth) or broken teeth. However, these materials hardly adhere to living tooth tissues. Therefore, for the purpose of bonding the restorative materials to living tooth tissues, dental adhesive compositions have been proposed which contain, as adhesive components, compounds having various polar groups (such as phosphoric acid groups, hydroxyl groups and acid anhydride groups) with an aim to improve the interaction with the main inorganic component such as apatite (calcium phosphate) or the organic component such as collagen constituting the tooth tissues.

However, with such a conventional technique, no bonding properties with the living tooth tissues, particularly with a dentin, have been observed. This is due to the physical and chemical structural factors of the living dentin. Namely, the dentin has a structure in which numerous dental tubeles are present and a body fluid is filled therein. Further, the proportion of protein such as collagen is relatively high in the dentin as compared with the enamel. Thus, the dentin has an environment extremely adverse to bonding. Accordingly, with the above mentioned conventional adhesive, it is intended to obtain certain adhesiveness to the living dentin by a so-called anchor effect by conducting pretreatment of apatite as the main inorganic component, with an etching agent such as phosphoric acid. Even then, the adhesive strength is still inadequate, and it happens that the restorative materials fall off after expiration of a long period of time. Thus, it can hardly be said that such an adhesive provides practically adequately high adhesive properties.

Further, the conventional adhesive contains a radical polymerizable unsaturated monomer as the main component. With such a monomer, it is usual that an unreacted unsaturated monomer is likely to remain even after the polymerization and curing. If it remains at a position where it is in direct contact with the dental pulp for a long period of time, it gives irritation to the dental pulp and causes inflammation, such being clinically undesirable.

It is an object of the present invention to provide a novel dental adhesive which does not require cumbersome pretreatment with an etching agent such as phosphoric acid which used to be required by the conventional method for bonding restorative materials to living tooth tissues, particularly to the dentin and which yet provides practically adequate adhesive strength and gives no irritation to the dental pulp.

The present invention provides a dental adhesive consisting essentially of a combination of:

(A) at least one trifunctional isocyanate compound, or a composition having an inert diluent incorporated to such isocyanate compound, and

(B) a mixture comprising, as the main components, (a) at least one radical polymerizable unsaturated monomer, (b) a radical polymerization initiator, and (c) a polymerizable phosphate compound.

Now, the present invention will be described in detail with reference to the preferred embodiments.

The trifunctional isocyanate compound of component (A) of the present invention is meant for a compound having three isocyanate groups, which can be obtained by reacting 3 mols of a diisocyanate compound to 1 mol of trimethylolpropane or to 1 mol of trimethylolethane.

The diisocyanate compound to be used here includes, for example, tolylene diisocyanate, 4,4'-diphenylmethane diisocyanate, hexamethylene diisocyanate, tetramethylene diisocyanate, xylene diisocyanate and isophorone diisocyanate.

The trifunctional isocyanate compound may be used alone. However, in order to improve the handling efficiency at the time of using the composition, a diluent inert to the isocyanate group, such as acetone, methyl ethyl ketone, ethyl acetate, toluene, xylene or trichloroethane, may be added.

With the dental adhesive of the present invention, basically, the essential bond strength can be obtained by component (A) only. However, for example, in a case where the bonding operation in the oral cavity is intended, there will be a time limitation for the curing of the adhesive layer, in many cases. Therefore, components (a), (b) and (c) constituting component (B) are combined to component (A) to overcome the above inconvenience. Namely, component (A) i.e. a trifunctional isocyanate compound or a mixture of a such a compound with a diluent, is coated on tooth tissues so that a film is formed on the surface of the tissues to form a dental pulp protective coating, and then a mixture of components (a), (b) and (c) is coated on the protective coating and polymerized for curing simultaneously at the time of curing the restorative material packed thereon. The radical polymerizable unsaturated monomer as component (a) of the present invention is a component which is necessary for quickly solidifying the adhesive layer by the radical polymerization initiator of component (b), and it may be any radical polymerizable unsaturated monomer, so

long as it does not adversely affect the adhesive properties of component (A). It may be of a mono-functional type or a poly-functional type. Here, the mono-functional unsaturated monomer and the polyfunctional unsaturated monomer are meant for an unsaturated monomer having one polymerizable unsaturated group and an unsaturated monomer having a plurality of polymerizable unsaturated groups, respectively.

Specific examples of the mono-functional unsaturated monomer include, methyl (meth)acrylate, ethyl (meth)acrylate, propyl (meth)acrylate, butyl (meth)acrylate, pentyl (meth)acrylate, hexyl (meth)acrylate, 2-hydroxyethyl (meth)acrylate, glycidyl (meth)acrylate, and benzyl (meth)acrylate. Among them, methyl methacrylate, benzyl methacrylate and 2-hydroxyethyl methacrylate are preferably used.

Specific examples of the bifunctional unsaturated monomer include polyethylene glycol di(meth)-acrylates represented by the following formula (1)

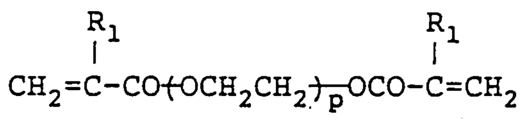

(1)

wherein each $R_1$ is a hydrogen atom or a methyl group independently, and p is an integer of from 1 to 20. Specific examples include ethylene glycol di(meth)acrylate, diethylene glycol di(meth)acrylate, triethylene glycol di(meth)acrylate, tetraethylene glycol di(meth)acrylate, pentaethylene glycol di(meth)acrylate, hexaethylene glycol di(meth)acrylate, heptaethylene glycol di(meth)acrylate, octaethylene glycol di(meth)-acrylate, nonaethylene glycol di(meth)acrylate, decaethylene glycol di(meth)acrylate, a polyethylene glycol di(meth)acrylate with a number of ethylene glycol unit (p) being 14, a polyethylene glycol di(meth)acrylate with p being 17, and a polyethylene glycol di(meth)acrylate with p being 19. Among them, ethylene glycol di(meth)acrylate, triethylene glycol di(meth)acrylate, nonaethylene glycol di(meth)acrylate and a polyethylene glycol di(meth)acrylate with p being 14, are preferably employed.

Further, 1,6-hexandiol di(meth)acrylate, neopentyl glycol di(meth)acrylate and trimethylolpropane tri-(meth)acrylate may also be used. Among them, trimethylolpropane triacrylate or 1,6-hexandiol di(meth)-acrylate is preferably employed.

Furthermore, bisphenol A type (meth)acrylates represented by the following formula (2):

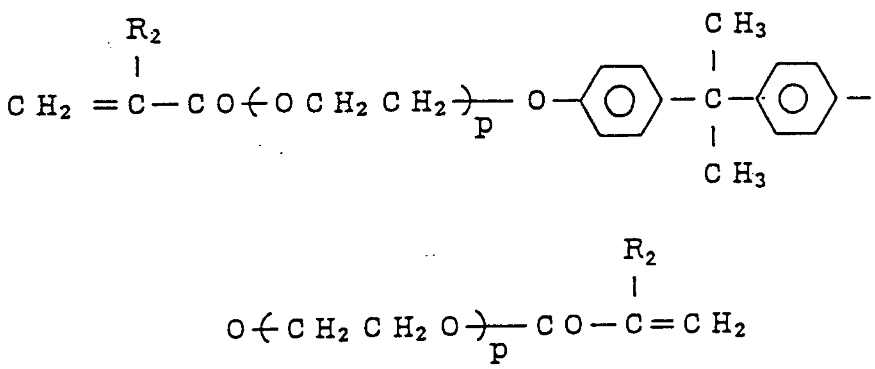

(2)

(wherein each $R_2$ represents H or a methyl group independently, and p is an integer of from 1 to 20) and of the following formula (3):

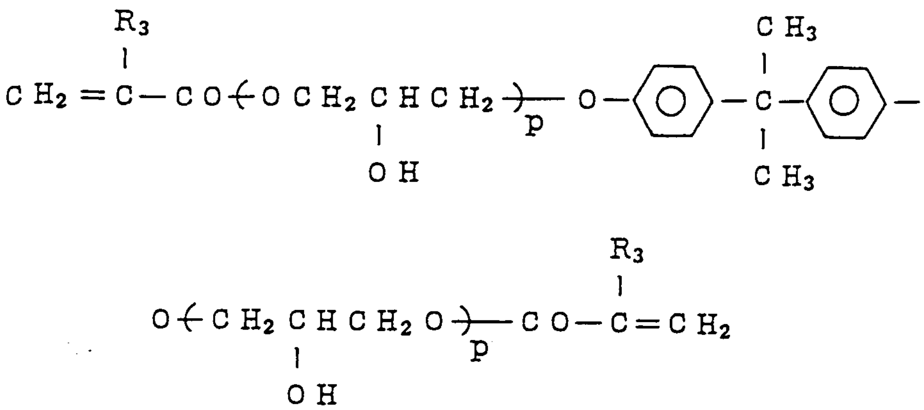

(3)

(wherein each $R_3$ is H or a methyl group independently, and p is an integer of from 1 to 20) may suitably be used. Specific examples thereof include 2,2'-bis(4-methacryloyloxypolyethoxyphenyl)propane, and 2,2'-bis[4-(3-methacryloyloxy-2-hydroxypropoxy)phenyl]propane. Further, 1,2-bis[3-(meth)acryloyloxy-2-hydroxypropoxy]ethane, and 1,4-bis[3-(meth)acryloyloxy-2-hydroxypropoxy]butane may also suitably be used.

Further, tetra-functional urethane(meth)acrylates represented by the formula (4):

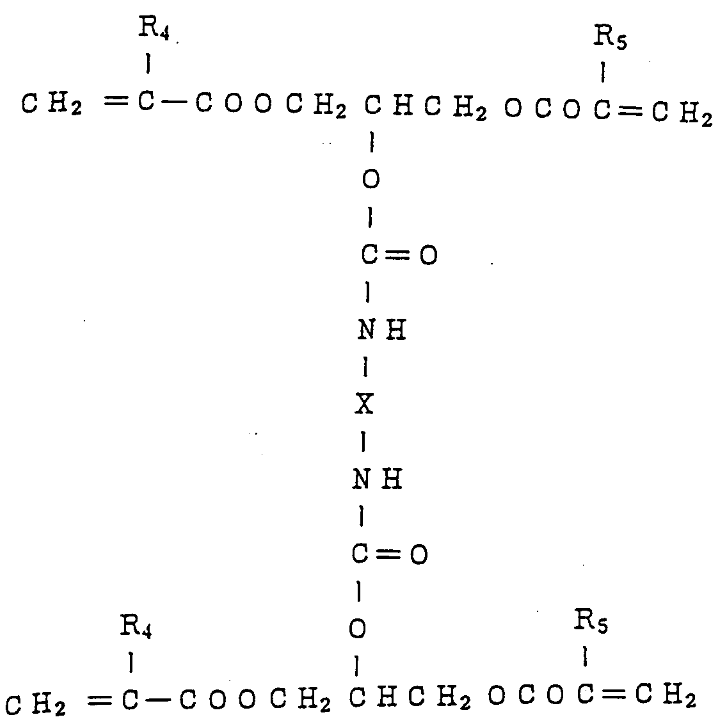

(4)

(wherein each of $R_4$ and $R_5$ is H or a methyl group, X is $-CH_2-$, $-CH_2CH_2-$, $-(CH_2)_3-$, $-(CH_2)_4-$, $-(CH_2)_5-$, $-(CH_2)_6-$ or

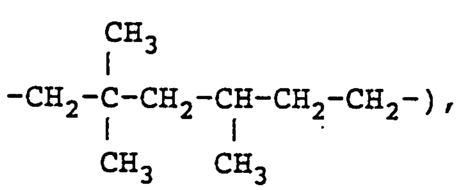

may also be employed. As specific examples thereof, a tetra-functional urethane(meth)acrylate of the formula (4) wherein $R_4$ is a hydrogen atom, $R_5$ is a methyl group and X is $-(CH_2)_6-$(hereinafter referred to simply as U-4HA) and a tetra-functional methacrylate of the formula (4) wherein each of $R_4$ and $R_5$ is a methyl group and X is $-(CH_2)_6-$(hereinafter referred to simply as U-4H) are preferably employed.

A further stronger adhesive strength can be obtained by using a polymerizable phosphate compound as component (c) of the present invention in combination with component (A) and component (a).

Specific examples of such a polymerizable phosphate include:

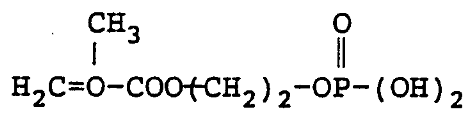

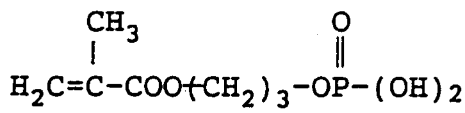

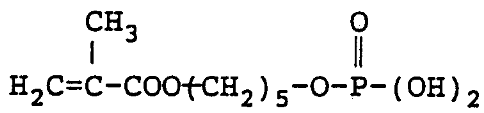

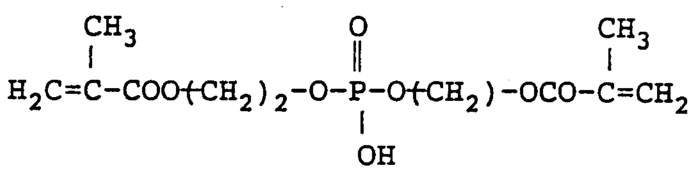

Among them, methacryloyloxyethyl phosphate and dimethacryloyloxyethyl phosphate are preferred.

The polymerizable phosphate compound is incorporated usually in an amount of from 0.1 to 30 parts by weight, preferably from 0.5 to 10 parts by weight, more preferably from 1.0 to 5.0 parts by weight, relative to the total radical polymerizable unsaturated monomer.

The radical polymerization initiator of component (b) of the present invention may be a photopolymerization initiator or a redox polymerization initiator.

As the photopolymerization initiator, a conventional ultraviolet polymerization initiator (such as benzophenone) or a conventional visible ray polymerization initiator may be used depending upon the particular purpose of its use. When it is to be used in an oral cavity, however, a photopolymerization initiator capable of initiating the polymerization by visible light within a wavelength range of from about 400 to 1,200 nm excluding the near ultraviolet region is preferably employed taking an adverse effect into consideration. Accordingly, a photosensitizer in the polymerization initiator is preferably the one excitable by light of a wavelength within a range of from about 400 to about 1,200 nm. As an example of such a compound, an $\alpha$-diketone compound may be mentioned. Specific examples of such an $\alpha$-diketone include, camphorquinone, benzil and diacetyl. Among them, camphorquinone is preferred, since it exhibits particularly high polymerization activities.

For the visible ray polymerization initiator of the present invention, it is preferred to use such photosensitizer in combination with a reducing agent such as a tertiary amine to obtain the desired excellent photopolymerization properties. Specific examples of such a tertiary amine include aliphatic amines such as trimethylamine, triethylamine and tripropylamine and aromatic amines such as isoamyl 4-(N,N-dimethylamino)benzoate, hexyl 4-(N,N-dimethylamino)benzoate, heptyl 4-(N,N-dimethylamino)benzoate, octyl 4-(N,N-dimethylamino)benzoate, 4,4'-bis(diethylamino)benzophenone and 4,4'-bis(dibutylamino)-benzophenone. Among them, aromatic tertiary amines are preferably employed. Particularly, 4,4'-bis-(diethylamino)benzophenone is most preferred, since it exhibits excellent visible ray polymerization properties when used in combination with camphorquinone. In general, the amount of photosensitizer is preferably within a range of from 0.01 to 30% by weight, and the amount of reducing agent is preferably within a range of from 0.01 to 30% by weight.

As to the amount of such a visible ray polymerization initiator to be added, an optimam amount exists depending upon the types of the photosensitizer and the reducing agent to be used. For example, in a system of camphorquinone and 4,4'-bis(diethylamino)benzophenone, the amount of camphorquinone is

preferably within a range of from 0.005 to 30% by weight, more preferably within a range of from 0.03 to 20% by weight, relative to the total radical polymerizable unsaturated monomers, and the amount of 4,4'-bis(diethylamino)benzophenone is preferably within a range of from 0.01 to 25% by weight, more preferably within a range of from 0.05 to 20% by weight.

As the redox polymerization initiator, a combination of an amine with a peroxide, a sulfinate with a peroxide, or an amine and a sulfinate with a peroxide, may suitably be employed.

As the peroxide, a conventional radical polymerization catalyst such as diacetyl peroxide, dilauroyl peroxide, distearoyl peroxide, dibenzoyl peroxide or a di-p-chlorobenzoyl peroxide, may be employed. Dibenzoyl peroxide is particularly preferred, since it is excellent in providing room temperature polymerizability.

As the amine, any of a primary amine, a secondary amine or a tertiary amine may be used. However, from the viewpoint of room temperature curing, a tertiary aromatic amine is preferably employed.

Specific examples of such preferred tertiary aromatic amine include N,N-dimethylaniline, N,N-diethylaniline, N,N-di($\beta$-hydroxyethyl)aniline, N,N-dimethyl-p-toluidine, N,N-diethyl-p-toluidine, N,N-di($\beta$-hydroxyethyl)-p-toluidine, N-methylaniline, N-methyl-p-toluidine, N,N-dimethylanisidine, N,N-diethylanisidine and diphenylamine. However, N,N-dimethyl-p-toluidine and N,N-di($\beta$-hydroxyethyl)-p-toluidine are particularly preferred, since when combined with a peroxide, they provide excellent room temperature polymerizability.

As the sulfinate, sodium p-toluene sulfinate, sodium benzenesulfinate, potassium benzenesulfinate, calcium benzenesulfinate, barium benzenesulfinate and ammonium benzenesulfinate, may be used. Among them, sodium p-toluenesulfinate is particularly preferred, since when combined with a peroxide, it provides excellent room temperature polymerizability.

Such a redox polymerization initiator is preferably added so that the peroxide will be within a range of from 0.01 to 10% by weight, more preferably within a range of from 0.05 to 5% by weight, relative to the total radical polymerizable unsaturated monomers, and the aromatic amine or sulfinate will be within a range of from 0.01 to 10% by weight, more preferably within a range of from 0.05 to 5% by weight.

The dental adhesive of the present invention consists essentially of a combination of the above component (A) and component (B) comprising components (a), (b) and (c). If necessary, however, an inorganic filler (such as silica powder, quartz powder or various glass powders), an organic polymer (such as polymethyl methacrylate or polystyrene), a coloring agent, a polymerization inhibiter (such as hydroquinone or 2,6-di-tert-butyl-4-mehtylphenol), an antioxidant, an ultraviolet absorber, a pigment, a dyestuff, etc. may be incorporated to component (B). The dental adhesive of the present invention is usually conveniently stored in two separate packages of component (A) and component (B) i.e. a mixture of components (a), (b) and (c) or such a mixture having the above mentioned further additives incorporated, until its practical use. However, in a case where a redox polymerization initiator is used as component (b), component (a) having component (c) incorporated therein, may be divided into two portions, and a peroxide may be incorporated to one portion, and an aromatic amine or a sulfinate may be incorporated to the other portion, so that they may be separately stored, and at the time of use, these two portions may be mixed and used as component (B).

Further, in the present invention, it is possible that the redox polymerization initiator and the photopolymerization initiator are used in combination.

The dental adhesive of the present invention can be applied to various restorative materials, and it is capable of providing excellent adhesive properties when applied not only to a thermosetting material such as a composite resin (a composite material having an inorganic filler incorporated to a poly-functional monomer) or a resin for crowns, which is commonly employed as a prosthetic material in this field, but also to a thermoplastic resin such as polymethyl methacrylate, polysulfone or polycarbonate, which is used as a resin for denture, or to various cement materials, amalgam, alumina, gold or an alloy material.

With the dental adhesive of the present invention, practically adequate adhesive strength can be obtained for the bonding of the restorative materials to the living tooth tissues, particularly to the dentin, without requiring cumbersome pretreatment with an etching agent such as phosphoric acid which used to be essential to the prior art.

Now, the present invention will be described in further detail with reference to Examples. However, it should be understood that the present invention is by no means restricted by such specific Examples.

Preparation of restorative material A (visible ray curable composite resin)

| | |
|---|---|
| 2,2-Bis[4-(methacryloyloxy ethoxy)phenyl]propane (hereinafter referred to simply as Bis-MEPP) | 8 g |
| Triethylene glycol dimethacrylate (hereinafter referred to simply as 3G) | 12 g |
| Silane-modified quartz powder (average particle size: about 4 $\mu$m) | 74 g |
| Silicon dioxide fine powder (#R-972, manufactured by Nippon Aerosil Co.) | 6 g |
| Camphorquinone | 0.4 g |
| Isoamyl 4-(N,N-dimethylamino)benzoate | 2 g |

A mixture (a paste) comprising the poly-functional monomer, the inorganic filler and the visible ray polymerization initiator having the above identified composition, i.e. a visible ray curable composite resin, was prepared in a dark room to obtain restorative material A.

Preparation of restorative material B (visible ray curable crown resin)

| | |
|---|---|
| 2,2-Bis[4-(3-methacryloyloxy-2-hydroxypropoxyphenyl]propane (hereinafter referred to simply as Bis-GMA) | 40 g |
| 3G | 60 g |
| Camphorquinone | 0.7 g |
| Isoamyl 4-(N,N-dimethylamino)benzoate | 2.8 g |

A mixture comprising the poly-functional monomer and the visible ray polymerization initiator having the above identified composition, i.e. a visible ray curable crown resin, was prepared in a dark room to obtain restorative material B.

Procedure for evaluating the adhesive properties and method for measuring the adhesive bond strength

(1) A fresh bovine anterior tooth immediately after extraction, was cut by a precision cutter (Isomet, manufactured by B hler Company) to expose the surface of the dentin, and the exposed flat surface was thoroughly polished with a water resistant polishing paper No. 1,000 under running water.
(2) As a dental adhesive, the one comprising the above mentioned components (A) and (B) was used. Firstly, component (A) was coated on the flat surface of the dentin to form a dental pulp protective coating. In a case where a diluent or the like is contained in component (A), an air stream is blown for above 10 seconds immediately after coating to evaporate and dissipate the diluent or the like. In a case where a diluent is contained in component (A), the coating film will be formed simply by evaporating and

dissipating the diluent. In a case where such a diluent is not contained, the coating film will be formed by leaving the coated surface to stand still for from 1 to 5 minutes after the coating. After the formation of the coating film, component (B) was applied.

(3) On the flat surface coated with the dental adhesive, a cylindrical silicon ring (one side openable) having an inner diameter of about 5 mm, a height of about 5 mm and a wall thickness of about 3 mm, was placed. Then, a liquid restorative material was filled in this silicon ring to a height of about 3 mm.

(4) To the upper end of the silicon ring filled with the restorative material, the emitting end of a visible ray irradiating device (GC light, manufactured by G C Dental Industry Co.) was brought in contact, and visible light was irradiated for 60 seconds to cure the restorative material.

(5) Upon expiration of about 10 minutes, the silicon ring was removed to obtain a bonded specimen.

(6) The entire test specimen was stored in water at 37°C for a predetermined period of time. Then, on the top of the restorative material, a spacer for bonding test (a rod of acrylic resin having the same diameter as the cured product of the adhesive) was bonded by means of a quickly polymerizable resin (Unifirst, manufactured by G C Dental Industry Co.), and then the specimen was set on a predetermined test jig, whereupon the tensile test was conducted, and the bond strength was measured. The conditions for the measurement are shown below.

Tensile tester: Tensilone, manufactured by Toyo Boldwin Company
Cross head speed (pulling speed): 0.5 mm/min
Full scale: 20 kgw

Synthesis of a trifunctional isocyanate compound

It was obtained by reacting 3 mols of a diisocyanate compound to 1 mol of trimethylolpropane or to 1 mol of trimethylolethane.

As the diisocyanate compound, tolylene diisocyanate (hereinafter referred to simply as TDI), 4,4'-diphenylmethane diisocyanate (hereinafter referred to simply as MDI) and hexamethylene diisocyanate (hereinafter referred to simply as HDI) were used.

Abbreviations for the trifunctional isocyanate compounds thus prepared are shown in Table 1.

Table 1

| Abbreviations of trifunctional isocyanate compounds | Polyols (1 mol) | Diisocyanates (3 mol) |
|---|---|---|
| PT | trimethylolpropane | TDI |
| PM | trimethylolpropane | MDI |
| PH | trimethylolpropane | HDI |
| ET | trimethylolethane | TDI |
| EM | trimethylolethane | MDI |
| EH | trimethylolethane | HEI |

Preparation of mixtures of radical polymerizable unsaturated monomer•visible ray photopolymerization initiator

Mixtures of various radical polymerizable unsaturated monomers and visible ray photopolymerization initiators, were prepared in a dark room by means of a general purpose mixer. In Table 2, the compositions and abbreviations of such mixtures are identified.

Table 2

| Abbreviations of mixtures of radical polymerizable unsaturated monomer·visible ray polymerization initiator | Radical polymerizable unsatureted monomers and their amounts | | | | |
|---|---|---|---|---|---|
| | Benzyl methacrylate | 2-Hydroxyethyl methacrylate | Triethylene glycol dimethacrylate | Bis-GMA | Bis-MEPP |
| LC-1 | - | - | 40 | 60 | - |
| LC-2 | - | - | 40 | 60 | - |
| LC-3 | 20 | - | 20 | - | 60 |
| LC-4 | 20 | - | 20 | - | 60 |
| LC-5 | - | 40 | - | - | - |
| LC-6 | - | 40 | - | - | - |
| LC-7 | - | - | - | 60 | 20 |
| LC-8 | - | - | - | 60 | 20 |
| LC-9 | - | 30 | 10 | 30 | - |
| LC-10 | - | 30 | 10 | 30 | - |

Table 2 (continued)

| Abbreviations of mixtures of radical polymerizable unsaturated monomer·visible ray polymerization initiator | Radical polymerizable unsatureted monomers and their amounts | | | Visible ray polymerization initiators and their amounts | |
|---|---|---|---|---|---|
| | U-4HA | Methacryloyl-oxyethyl phosphate | Dimethacryloyl-oxyethyl phosphate | Camphorquinone | 4,4-Bis(diethylamino) benzophenone |
| LC-1 | - | 2.0 | - | 0.8 | 1.2 |
| LC-2 | - | - | 2.0 | 0.8 | 1.2 |
| LC-3 | - | 2.0 | - | 0.8 | 1.2 |
| LC-4 | - | - | 2.0 | 0.8 | 1.2 |
| LC-5 | 60 | 2.0 | - | 0.8 | 1.2 |
| LC-6 | 60 | - | 2.0 | 0.8 | 1.2 |
| LC-7 | 20 | 2.0 | - | 1.2 | 1.6 |
| LC-8 | 20 | - | 2.0 | 1.2 | 1.6 |
| LC-9 | 30 | 2.0 | - | 1.2 | 1.6 |
| LC-10 | 30 | - | 2.0 | 1.2 | 1.6 |

Preparation of mixtures of radical polymerizable unsaturated monomer•redox polymerization initiator

Mixtures of various radical polymerizable unsaturated monomers and redox polymerization initiators were prepared by means of a general purpose mixer. The compositions and abbreviations of such mixtures

are shown below.

| Radical polymerizable unsaturated monomer•redox polymerization initiator mixture A: | |
|---|---|
| U-4HA | 20 g |
| Triethylene glycol dimethacrylate | 10 g |
| Bis-GMA | 50 g |
| 2-Hydroxyethyl methacrylate | 20 g |
| Methacryloyloxyethyl phosphate | 2.0 g |
| Dibenzoyl peroxide | 1.0 g |

| Radical polymerizable unsaturated monomer•redox polymerization initiator mixture B: | |
|---|---|
| 2-Hydroxyethyl methacrylate | 10 g |
| Ethanol | 9.8 g |
| Dihydroxyethyl-p-toluidine | 0.8 g |
| Sodium p-toluenesulfinate | 0.5 g |

Examples 1 to 6

Various trifunctional isocyanate compounds shown in Table 3 were used as component (A), and LC-9 i.e. the mixture of a radical polymerizable unsaturated monomer and a visible ray polymerization initiator, shown in Table 2, was used as component (B). In accordance with the procedure for evaluating the adhesive properties and the procedure for measuring the adhesive bond strength, the adhesive properties of restorative material A to the surface of the dentin of a bovine tooth by means of such adhesives without etching treatment were evaluated. The results are shown in Table 3.

Table 3

| Example No. | Adhesive components | | Average bond strength to the dentin ($kg/cm^2$) |
|---|---|---|---|
| | Trifunctional isocyanate compound | Radical polymerizable unsaturated monomer·visible ray polymerization initiator mixture | Storage in water at 37°C 1 day later |
| 1 | PT | LC-9 | 35.8 |
| 2 | PM | LC-9 | 54.2 |
| 3 | PH | LC-9 | 48.6 |
| 4 | ET | LC-9 | 41.5 |
| 5 | EM | LC-9 | 50.2 |
| 6 | EM | LC-9 | 54.6 |

Examples 7 to 12

The tests were conducted in the same manner as in Example 1 except that various trifunctional isocyanate compounds shown in Table 4 were used as component (A) and a mixture prepared by mixing equal amounts of the radical polymerizable unsaturated monomer•redox polymerization initiator mixture A and the radical polymerizable unsaturated monomer•redox polymerization initiator mixture B immediately prior to use, was used as component (B). The results are shown in Table 4.

Table 4

| Example No. | Adhesive components | | Average bond strength to the dentin (kg/cm$^2$) |
|---|---|---|---|
| | Trifunctional isocyanate compound | Radical polymerizable unsaturated monomer·redox polymerization initiator mixture | Storage in water at 37°C 1 day later |
| 7 | PT | Mixtures A and B | 26.3 |
| 8 | PM | Mixtures A and B | 34.0 |
| 9 | PH | Mixtures A and B | 42.1 |
| 10 | ET | Mixtures A and B | 40.6 |
| 11 | EM | Mixtures A and B | 35.8 |
| 12 | EM | Mixtures A and B | 37.4 |

Comparative Examples 1 to 10

With respect to adhesive compositions composed solely of various radical polymerizable unsaturated monomer•visible ray polymerization initiator mixtures, the adhesive properties of restorative material A were evaluated in the same procedure as in Example 1. The results are shown in Table 5.

Table 5

| Comparative Example No. | Adhesive components | | Average bond strength to the dentin ($kg/cm^2$) |
|---|---|---|---|
| | Trifunctional isocyanate compound | Radical polymerizable unsaturated monomer·visible ray polymerization initiator mixture | Storage in water at 37°C 1 day later |
| 1 | Not used | LC-1 | 0 |
| 2 | Not used | LC-2 | 0 |
| 3 | Not used | LC-3 | 0 |
| 4 | Not used | LC-4 | 0 |
| 5 | Not used | LC-5 | 0 |
| 6 | Not used | LC-6 | 0 |
| 7 | Not used | LC-7 | 0 |
| 8 | Not used | LC-8 | 0 |
| 9 | Not used | LC-9 | 0 |
| 10 | Not used | LC-10 | 0 |

Examples 13 to 16

In the same manner as in Example 3 except that restoration materials identified in Table 6 were used instead of restoration material A, the adhesive properties to various restorative materials were evaluated. The results are shown in Table 6.

Table 6

| Example No. | Restorative materials | Average bond strength to the dentin ($kg/cm^2$)*1 |
|---|---|---|
| 13 | Restorative Material B | 43.1 |
| 14 | Commercially available one pack type composite resin*2 | 38.6 |
| 15 | Commercially available two pack type composite resin*3 | 39.5 |
| 16 | Polymethyl methacrylate*4 | 47.3 |

*1: The bond strength was measured after storage in water at 37°C for one day using three specimens per test

*2: Occlusin, manufactured by ICI Company.

*3: Microrest AP, manufactured by G C Dental Industry Co.

*4: Acrypet #VH, manufactured by Mitsubishi Rayon Company Limited

Example 17

Clinical tests were conducted with respect to teeth (in oral cavities) after having carious portions removed from dental carries of 32 cases of 20 persons, instead of the bovine tooth used in Example 1. As the results, in all cases, no inflammation was observed at the dental pulp, and no pain was reported after the operation.

**Claims**

**1.** A dental adhesive consisting essentially of a combination of:
(A) at least one trifunctional isocyanate compound, or a composition having an inert diluent incorporated to such isocyanate compound, and
(B) a mixture comprising, as the main components, (a) at least one radical polymerizable unsaturated monomer, (b) a radical polymerization initiator, and (c) a polymerizable phosphate compound.

**2.** The dental adhesive according to Claim 1, wherein the trifunctional isocyanate compound is the one wherein 3 molar times of a diisocyanate is boned to trimethylol propane or trimethylol ethane by the reaction of one of the isocyanate groups of the diisocyanate with the methylol group.

**3.** The dental adhesive according to Claim 2, wherein the diisocyanate is selected from the group consisting of tolylene diisocyanate, 4,4'-diphenylmethane diisocyanate, hexamethylene diisocyanate, tetramethylene diisocyanate, xylene diisocyanate and isophorone diisocyanate.

**4.** The dental adhesive according to Claim 1, wherein the inert diluent is selected from the group consisting of acetone, methyl ethyl ketone, ethyl acetate, toluene, xylene and trichloroethane.

**5.** The dental adhesive according to Claim 1, wherein the radical polymerizable unsaturated monomer is the one having a (meth)acryloyloxy group.

**6.** The dental adhesive according to Claim 5, wherein the radical polymerizable unsaturated monomer is

at least one member selected from the group consisting of methyl (meth)acrylate, ethyl (meth)acrylate, propyl (meth)acrylate, butyl (meth)acrylate, pentyl (meth)acrylate, hexyl (meth)acrylate, 2-hydroxyethyl (meth)acrylate, glycidyl (meth)acrylate, benzyl (meth)acrylate, a polyethylene glycol di(meth)acrylate of the formula (1):

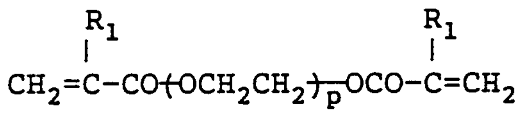

(1)

(wherein each $R_1$ is a hydrogen atom or a methyl group independently, and p is an integer of from 1 to 20), 1,6-hexanediol di(meth)acrylate, neopentyl glycol di(meth)acrylate, trimethylolpropane tri(meth)-acrylate, dimethacryloyloxyethyl phosphate, a bisphenol A type di(meth)acrylate of the formula (2) or (3):

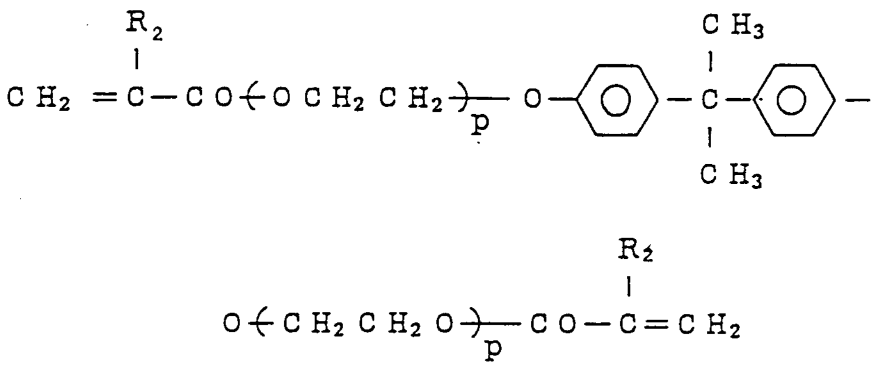

(2)

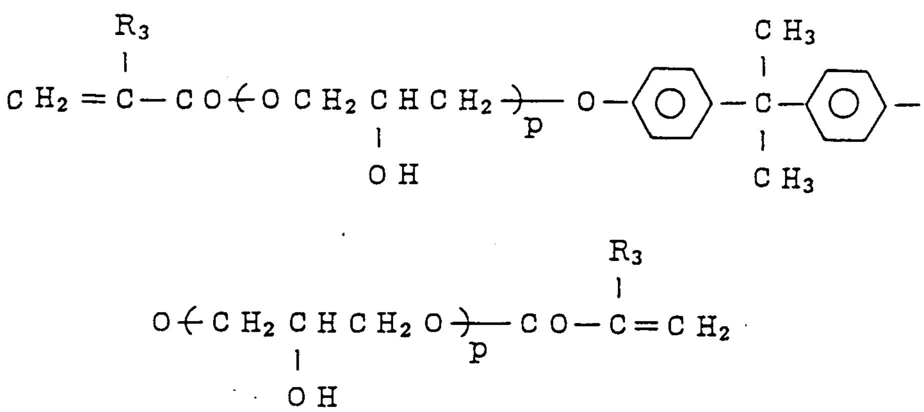

(3)

(wherein each of $R_2$ and $R_3$ represents a hydrogen atom or a methyl group independently, and p is an integer of from 1 to 20), 1,2-bis[3-(meth)acryloyloxy-2-hydroxypropoxy]ethane, 1,4-bis[3-(meth)-acryloyloxy-2-hydroxypropoxy]butane, and a tetra-functional urethane (meth)acrylate of the formula (4):

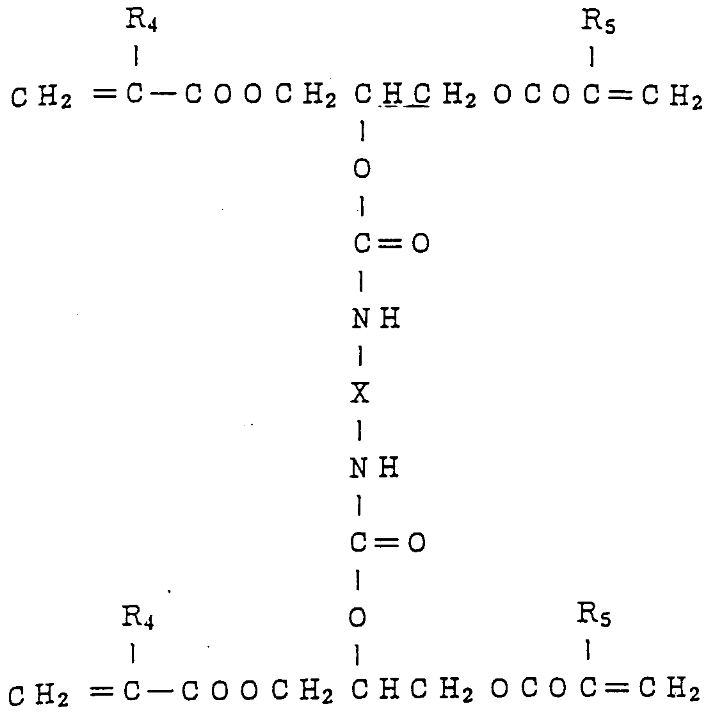

(4)

(wherein each of $R_4$ and $R_5$ represents a hydrogen atom or a methyl group independently, and X is $-CH_2-$, $CH_2CH_2-$, $-(CH_2)_3-$, $-(CH_2)_4-$, $-(CH_2)_5-$, $-(CH_2)_6-$ or

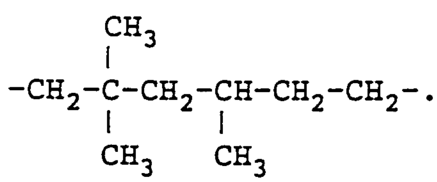

7. The dental adhesive according to Claim 1, wherein the polymerizable phosphate compound is at least one member selected from the group consisting of methacryloyloxyethyl phosphate, methacryloyloxypropyl phosphate, methacryloyloxypentyl phosphate, and di(methacryloyloxyethyl) phosphate.

European Patent Office

# EUROPEAN SEARCH REPORT

Application Number

**EP 90 11 4400**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 301 516 (MISUBSIHI)<br>* page 3, line 29 - page 13, line 35; claims 1, 2, 11-24 *example 247-249 *<br>– – – | 1-7 | A 61 K 6/00<br>C 08 G 18/80 |
| A | EP-A-0 363 903 (DENTSPLY)<br>* page 4, line 11 - page 8, line 15 *<br>– – – | 1-7 | |
| A | EP-A-0 321 683 (DENTSPLY)<br>* page 3, line 37 - page 5, line 27; claims 1-14 *<br>– – – | 1,7 | |
| A | EP-A-0 195 224 (IVOCLAR)<br>* page 3, line 20 - page 6, line 19; claims 1-10 * *examples *<br>– – – | 1-3 | |
| A | US-A-4 134 935 (QUIRING ET AL)<br>– – – – – | | |

TECHNICAL FIELDS SEARCHED (Int. Cl.5)

A 61 K
C 08 G

The present search report has been drawn up for all claims

| Place of search | Date of completion of search | Examiner |
|---|---|---|
| The Hague | 01 March 91 | BOURGONJE A.F. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document